# EUROPEAN PATENT APPLICATION

(11) **EP 4 296 336 A1**
(43) Date of publication of application: **27.12.2023**
(21) Application number: 22907327.5
(22) Date of filing: 07.12.2022
(51) Int. Cl.: C10G 2/00, B01D 53/22, C07C 29/152, C07C 31/04, C07C 31/08

(54) **LIQUID FUEL SYNTHESIS SYSTEM AND LIQUID FUEL SYNTHESIS METHOD**

(30) Priority: 17.12.2021 JP 2021204940; 28.04.2022 JP 2022074719
(71) Applicant: NGK Insulators, Ltd., Nagoya-shi, Aichi 467-8530 (JP)
(72) Inventor: KAN, Hirofumi, Nagoya-shi, Aichi 467-8530 (JP); TORII, Atsushi, Nagoya-shi, Aichi 467-8530 (JP); NAKAGAWA, Kosuke, Nagoya-shi, Aichi 467-8530 (JP); IIDA, Kazuki, Nagoya-shi, Aichi 467-8530 (JP)
(74) Representative: Müller-Boré & Partner Patentanwälte PartG mbB
(86) International application number: PCT/JP2022/045151
(87) International publication number: WO 2023/112800

(57) **Abstract**

A liquid fuel synthesis system (100) includes a liquid fuel synthesis unit (110) and a sweep gas supply unit (120). The liquid fuel synthesis unit (110) is permeable to water vapor, which is a product of a conversion reaction of a raw material gas containing at least hydrogen and carbon dioxide to a liquid fuel. The sweep gas supply unit (120) supplies a sweep gas for sweeping the water vapor permeating through the separation membrane (112) to the liquid fuel synthesis unit (110). The sweep gas contains hydrogen or carbon dioxide as a main component.

## Description

### TECHNICAL FIELD

The present invention relates to a liquid fuel synthesis system and a liquid fuel synthesis method.

### BACKGROUND ART

In recent years, liquid fuel synthesis systems have been developed that can improve, in a conversion reaction of a raw material gas containing hydrogen and carbon dioxide to a liquid fuel such as methanol and ethanol (specifically, a fuel that is in a liquid state at normal temperature and pressure), conversion efficiency by separating water vapor which is a by-product.

Patent Literature 1 discloses a liquid fuel synthesis system including a membrane reactor, a raw material gas supply unit, and a sweep gas supply unit. The membrane reactor includes a catalyst for promoting the conversion reaction of a raw material gas containing hydrogen and carbon dioxide to methanol, and a separation membrane permeable to water vapor which is a by-product of the conversion reaction. The raw material gas supply unit supplies the raw material gas to a non-permeation side of the separation membrane. The sweep gas supply unit supplies a sweep gas to a permeation side of the separation membrane. Water vapor that has permeated through the separation membrane is discharged from the membrane reactor together with the sweep gas.

### CITATION LIST

### Patent Literature

Patent Literature 1: JP 2018-008940A

### SUMMARY

### TECHNICAL PROBLEM

Here, since the molecular diameters of water and hydrogen are close to each other, some of the hydrogen contained in the raw material gas is likely to permeate through the separation membrane and get mixed with the sweep gas. Therefore, if the hydrogen mixed with the sweep gas can be reused, utilization efficiency of the raw material gas can be improved.

However, since at least one of nitrogen and air is used as the sweep gas in the liquid fuel synthesis system disclosed in Patent Literature 1, in order to reuse the hydrogen mixed with the sweep gas, the hydrogen needs to be individually separated, which is complicated.

This invention has been made in view of the above-described circumstances, and aims to provide a liquid fuel synthesis system and a liquid fuel synthesis method that can improve utilization efficiency of a raw material gas.

### SOLUTION TO PROBLEM

A liquid fuel synthesis system according to a first aspect of the present invention includes a liquid fuel synthesis unit and a sweep gas supply unit. The liquid fuel synthesis unit is permeable to a product of a conversion reaction of a raw material gas containing at least hydrogen and carbon dioxide to a liquid fuel. The sweep gas supply unit for supplying a sweep gas for sweeping the product has permeating through the separation membrane to the liquid fuel synthesis unit. The sweep gas contains hydrogen or carbon dioxide as a main component.

The liquid fuel synthesis system according to a second aspect of the present invention is in accordance with the first aspect, and the sweep gas contains hydrogen as a main component.

The liquid fuel synthesis system according to a third aspect of the present invention is in accordance with the second aspect, and the sweep gas contains carbon dioxide as a secondary component.

The liquid fuel synthesis system according to a fourth aspect of the present invention is in accordance with one of the first to third aspects, and includes a moisture removal unit for removing moisture from a discharged gas discharged from the liquid fuel synthesis unit, the discharged gas containing the sweep gas and the product.

The liquid fuel synthesis system according to a fifth aspect of the present invention is in accordance with the fourth aspect, and the moisture removal unit includes a heat exchanger using a material gas containing at least hydrogen and carbon dioxide, as a refrigerant.

The liquid fuel synthesis system according to a sixth aspect of the present invention is in accordance with the fifth aspect, and includes a pressurizing unit for pressurizing a mixed gas in which the material gas passing through the moisture removal unit and the sweep gas are mixed. The pressurizing unit is configured to supply the mixed gas to the liquid fuel synthesis unit.

The liquid fuel synthesis method according to a seventh aspect of the present invention includes a step of supplying a sweep gas for sweeping a product to a permeation side of a separation membrane while promoting a conversion reaction of a raw material gas containing at least hydrogen and carbon dioxide to a liquid fuel by supplying the raw material gas to a non-permeation side of the separation membrane, the product being generated through the conversion reaction and permeating through the separation membrane. The sweep gas contains hydrogen or carbon dioxide as a main component.

The liquid fuel synthesis system according to an eighth aspect of the present invention is in accordance with the seventh aspect, and further includes a step of removing moisture from a discharged gas containing the sweep gas and the product.

The liquid fuel synthesis system according to a ninth aspect of the present invention is in accordance with the eighth aspect, and in the step of removing moisture from the discharged gas, a material gas containing at least hydrogen and carbon dioxide is used as a refrigerant.

The liquid fuel synthesis system according to a tenth aspect of the present invention is in accordance with the ninth aspect, and further includes a step of pressurizing a mixed gas in which the material gas used as the refrigerant and the sweep gas are mixed.

### ADVANTAGEOUS EFFECTS

According to the present invention, a liquid fuel synthesis system and a liquid fuel synthesis method capable of improving utilization efficiency of a raw material gas can be provided.

### BRIEF DESCRIPTION OF DRAWINGS

FIG. 1 is a schematic view showing a configuration of a liquid fuel synthesis system according to an embodiment.
FIG. 2 is a schematic view showing a configuration of a liquid fuel synthesis system according to modification 2.
FIG. 3 is a schematic view showing a configuration of a liquid fuel synthesis system according to modification 5.
FIG. 4 is a schematic view showing a configuration of the liquid fuel synthesis system according to modification 5.
FIG. 5 is a schematic view showing a configuration of the liquid fuel synthesis system according to modification 5.
FIG. 6 is a schematic view showing a configuration of the liquid fuel synthesis system according to modification 5.

### DESCRIPTION OF EMBODIMENTS

Next, embodiments of the present invention will be described with reference to the drawings. However, the drawings are schematic, and ratio or the like of dimensions may differ from an actual one.

### Liquid Fuel Synthesis System

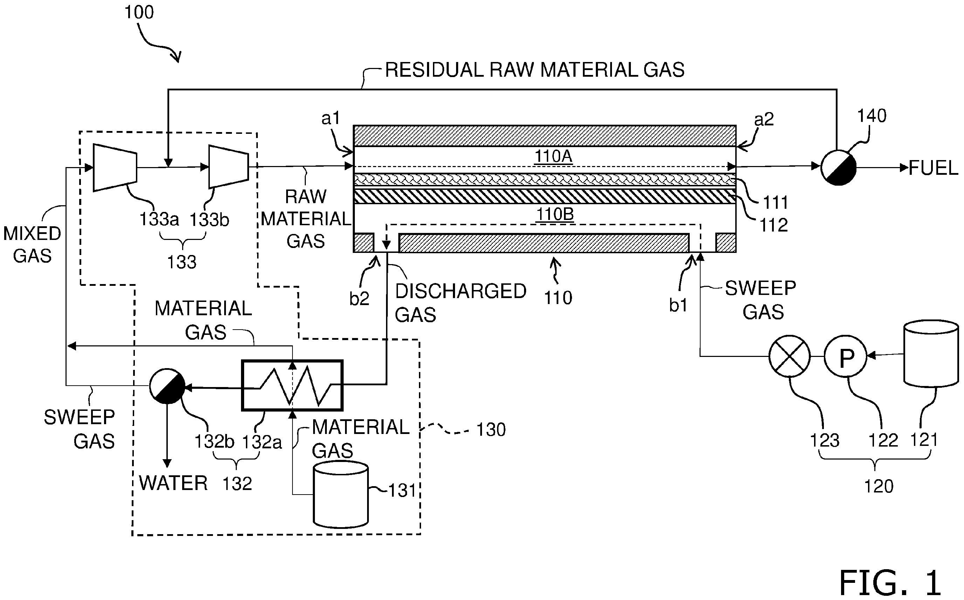

FIG. 1 is a schematic view showing a configuration of a liquid fuel synthesis system 100. The liquid fuel synthesis system 100 includes a liquid fuel synthesis unit 110, a sweep gas supply unit 120, a raw material gas supply unit 130, and a first drain trap 140.

The liquid fuel synthesis unit 110 is a so-called membrane reactor used to convert a raw material gas to a liquid fuel. The shape of the liquid fuel synthesis unit 110 is not particularly limited, and examples thereof may include a monolith shape, a planar shape, a tubular shape, a cylindrical shape, a columnar shape, and a polygonal columnar shape. A monolith shape means a shape including a plurality of cells that pass through in a longitudinal direction, and is a concept including a honeycomb shape.

The raw material gas is supplied from the raw material gas supply unit 130 to the liquid fuel synthesis unit 110. The raw material gas contains at least hydrogen and carbon dioxide. The raw material gas may contain carbon monoxide. The raw material gas may be a so-called synthetic gas (syngas). The liquid fuel is a fuel that is in a liquid state at normal temperature and pressure, or a fuel that can be liquidized at normal temperature and pressurized state. Examples of the liquid fuel that is in a liquid state at normal temperature and pressure may include methanol, ethanol, liquid fuels represented by CₙH2₍ₘ₋₂ₙ₎ (m is an integer less than 90, and n is an integer less than 30), and mixtures thereof. Examples of the fuel that can be liquidized at normal temperature and pressurized state include, for example, propane, butane, and mixtures thereof.

For example, reaction formula (1) for synthesizing methanol by catalytically hydrogenating a raw material gas containing carbon dioxide and hydrogen in the presence of a catalyst is as follows.

CO_{2 +} 3H₂ ⇔ CH₃OH ₊ H₂O (1)

The above reaction is an equilibrium reaction, and in order to increase both the conversion rate and the reaction rate, it is preferable to carry out the reactions at a high temperature and high pressure (for example, 180°C or higher and 2 MPa or higher). The liquid fuel is in a gaseous state when it is synthesized, and is kept in a gaseous state at least until it flows out of the liquid fuel synthesis unit 110. The liquid fuel synthesis unit 110 preferably has heat resistance and pressure resistance suitable for desired synthesis conditions of the liquid fuel.

The liquid fuel synthesis unit 110 according to the present embodiment has a catalyst layer 111, separation membrane 112, a non-permeation side space 110A, and a permeation side space 110B.

The catalyst layer 111 is disposed in the non-permeation side space 110A. A conversion reaction of the raw material gas to a liquid fuel is promoted in the catalyst layer 111.

The catalyst layer 111 is a porous body constituted by a porous material and a catalyst. An average pore diameter of the catalyst layer 111 can be 5 pm or more and 25 pm or less. The average pore diameter of the catalyst layer 111 can be measured using the mercury intrusion technique. The porosity of the catalyst layer 111 can be 25% or more and 50% or less. The average particle size of the porous material constituting the catalyst layer 111 can be 1 pm or more and 100 pm or less. In the present embodiment, the average particle size is an arithmetic mean value of maximum diameters of (randomly selected) 30 measurement target particles measured through cross-sectional microstructure observation using a Scanning Electron Microscope (SEM).

As the porous material, a ceramic material, a metal material, a resin material, or the like can be used, and a ceramic material is particularly preferable. As an aggregate of the ceramic material, alumina (Al₂O₃), titania (TiO₂), mullite (Al₂O₃-SiO₂), potsherd, cordierite (Mg₂Al₄Si₅O₁₈), or the like can be used, and alumina is preferable in consideration of availability, clay stability, and corrosion resistance. As an inorganic binder for the ceramic material, at least one of titania, mullite, readily sinterable alumina, silica, glass frit, a clay mineral, and readily sinterable cordierite can be used. However, the ceramic material does not need to contain an inorganic binder.

The catalyst promotes the conversion reaction of the raw material gas to the liquid fuel. The catalyst is disposed in pores of the porous material. The catalyst may be supported on inner surfaces of the pores. Alternatively, a carrier that supports the catalyst may adhere to the inner surfaces of the pores.

As the catalyst, a known catalyst suitable for the conversion reaction to a desired liquid fuel may be used. Specifically, metal catalysts (copper, palladium, and the like), oxide catalysts (zinc oxide, zirconia, gallium oxide, and the like), and composite catalysts thereof (copper-zinc oxide, copper-zinc oxide-alumina, copper-zinc oxide-chromium oxide-alumina, copper-cobalt-titania, catalysts obtained by modifying these with palladium, and the like) can be used.

The separation membrane 112 is permeable to water vapor, which is one of the products of the conversion reaction of the raw material gas to the liquid fuel. In this manner, by utilizing the equilibrium shift effect, the reaction equilibrium of the above formula (1) can be shifted to the product side.

The molecular diameter of water (0.26 nm) is close to the molecular diameter of hydrogen (0.296 nm). For this reason, in the present embodiment, it is assumed that not only water vapor, which is a product of the conversion reaction, but some of the hydrogen contained in the raw material gas permeates through the separation membrane 112.

The separation membrane 112 preferably has a water vapor permeability coefficient of 100 nmol/(s·Pa·m²) or more. The water vapor permeability coefficient can be determined using a known method (see Ind. Eng. Chem. Res., 40, 163-175 (2001)).

The separation membrane 112 preferably has a separation factor of 100 or more. The greater the separation factor is, the more permeable the separation membrane 112 is to water vapor, and less permeable to components other than water vapor (e.g., hydrogen, carbon dioxide, and liquid fuel). The separation factor can be determined using a known method (see FIG. 1 of "Separation and Purification Technology 239 (2020) 116533").

An inorganic membrane can be used as the separation membrane 112. The inorganic membrane is preferable because it has heat resistance, pressure resistance, and water vapor resistance. Examples of the inorganic membrane include a zeolite membrane, a silica membrane, an alumina membrane, and a composite membrane thereof. In particular, an LTA zeolite membrane having a molar ratio (Si/Al) of a silicon element (Si) and an aluminum element (Al) of 1.0 or more and 3.0 or less is suitable because of its excellent water vapor permeability.

Note that the separation membrane 112 may be supported by the porous substrate.

The non-permeation side space 110A is a space located on a non-permeation side of the separation membrane 112. The raw material gas is supplied from the raw material gas supply unit 130 to the non-permeation side space 110A. The raw material gas flows to the non-permeation side space 110A through an inflow port a1. A liquid fuel synthesized in the catalyst layer 111 flows out of the non-permeation side space 110A through an outflow port a2. The liquid fuel flowing out of the outflow port a2 may be mixed with unreacted residual raw material gas. The residual raw material gas mixed with the liquid fuel is separated from the liquid fuel in the first drain trap 140. The separated residual raw material gas is returned to the raw material gas supply unit 130 (specifically, a second booster pump 133b, which will be described later). The residual raw material gas contains at least one of hydrogen and carbon dioxide.

The permeation side space 110B is a space located on a permeation side of the separation membrane 112. The water vapor and hydrogen that have permeated through the separation membrane 112 flow to the permeation side space 110B. The sweep gas is supplied from the sweep gas supply unit 120 to the permeation side space 110B. The sweep gas flows to the permeation side space 110B through an inflow port b1. A discharged gas containing the sweep gas and water vapor flows out of the permeation side space 110B through an outflow port b2.

The sweep gas supply unit 120 is disposed on the upstream side of the permeation side space 110B. The sweep gas supply unit 120 includes a storage unit 121, a flow rate adjustment mechanism 122, and a heating unit 123.

The storage unit 121 stores the sweep gas. The sweep gas contains hydrogen or carbon dioxide as the main component. Thus, due to the sweep gas containing hydrogen or carbon dioxide as the main component, hydrogen permeating through the separation membrane 112 can be reused as part of the raw material gas without separating from the sweep gas. As a result, the utilization efficiency of the raw material gas can be easily improved. Note that containing hydrogen or carbon dioxide as the main component means having the highest content rate of hydrogen or carbon dioxide of gases contained in the sweep gas.

The sweep gas may contain only one of hydrogen and carbon dioxide, but may contain both hydrogen and carbon dioxide. If the sweep gas contains both hydrogen and carbon dioxide, the specific heat of the sweep gas can be increased compared to a case where the sweep gas contains only one of hydrogen and carbon dioxide, and therefore the removal efficiency of the heat generated along with the synthesis of the liquid fuel can be improved.

The sweep gas preferably contains hydrogen as the main component. Thus, the difference between a hydrogen partial pressure in the non-permeation side space 110A and a hydrogen partial pressure in the permeation side space 110B can be reduced, and therefore the amount of hydrogen permeating through the separation membrane 112 can be suppressed. The content ratio of hydrogen in the sweep gas is not particularly limited, but can be, for example, 60 mol% or more and 100 mol% or less.

The sweep gas preferably contains carbon dioxide as the secondary component. Thus, it is possible to prevent the dew point (that is, humidity) of the discharged gas from being reduced due to an excessive decrease in the ratio of the amount of exhaust gas to the amount of water in the exhaust gas. As a result, the load of a heat exchanger 132a (described later) can be reduced. Containing carbon dioxide as the secondary component means that the content ratio of carbon dioxide in gases contained in the sweep gas is the second highest after hydrogen. The content ratio of carbon dioxide in the sweep gas is not particularly limited, but can be, for example, 5 mol % or more and 40 mol % or less.

The flow rate adjustment mechanism 122 adjusts the flow rate of the sweep gas supplied from the storage unit 121. A pump, a blower, or the like can be used as the flow rate adjustment mechanism 122. Note that in the case where the sweep gas is stored in the storage unit 121 in the pressurized state, the flow rate adjustment mechanism 122 can be omitted.

A heating unit 123 heats the sweep gas to a desired temperature. There is particularly no limitation on the heating unit 123 as long as the heating unit 123 can heat the sweep gas. The heating unit 123 may heat the sweep gas using a reused heat exchanger using heat exchange with the heat exchanger 132a (described later).

The raw material gas supply unit 130 is disposed on the downstream side of the non-permeation side space 110A. The raw material gas supply unit 130 has a material gas source 131, a moisture removal unit 132, and a pressurizing unit 133.

The material gas source 131 stores material gas. The material gas contains at least hydrogen and carbon dioxide. The material gas may contain carbon monoxide. The material gas may be a so-called synthetic gas (syngas). The material gas stored in the material gas source 131 is supplied to the moisture removal unit 132.

The moisture removal unit 132 removes moisture from the discharged gas that is discharged from the liquid fuel synthesis unit and contains the sweep gas and water vapor. In this manner, the sweep gas is separated from the discharged gas. The moisture removal unit 132 has the heat exchanger 132a and a second drain trap 132b.

The heat exchanger 132a includes a first flow path c1 through which the material gas supplied from the material gas source 131 flows, and a second flow path c2 through which the discharged gas discharged from the liquid fuel synthesis unit 110 flows. The heat exchanger 132a condenses water vapor in the discharged gas to water by using the material gas as a refrigerant. In this manner, the material gas can be heated and the discharged gas can be cooled at the same time, and thus, heat efficiency in the liquid fuel synthesis system 100 can be improved.

The second drain trap 132b is disposed on the downstream side of the heat exchanger 132a. The second drain trap 132b separates the water obtained by condensation by the heat exchanger 132a from the sweep gas. The sweep gas separated by the second drain trap 132b is mixed with the material gas that has passed through the heat exchanger 132a, on the downstream side of the second drain trap 132b. In this manner, the mixed gas in which the sweep gas and the material gas are mixed is generated.

The mixed gas is supplied to the pressurizing unit 133. The pressurizing unit 133 is disposed on the downstream side of the second drain trap 132b and the upstream side of the liquid fuel synthesis unit 110. The pressurizing unit 133 pressurizes the material gas and the sweep gas that have passed through the moisture removal unit 132 and supplies the resultant gases to the liquid fuel synthesis unit 110. The pressurizing unit 133 includes a first booster pump 133a and a second booster pump 133b.

The first booster pump 133a boosts the mixed gas to a predetermined first pressure. The mixed gas boosted by the first booster pump 133a is mixed with the residual raw material gas separated from the liquid fuel in the first drain trap 140. In this manner, the raw material gas in which the mixed gas and the residual raw material gas are mixed is generated.

The second booster pump 133b boosts the raw material gas to a predetermined second pressure. The second pressure is a pressure suitable for the conversion reaction from the raw material gas to the liquid fuel, and is higher than the first pressure. The raw material gas boosted by the second booster pump 133b is supplied to the non-permeation side space 110A of the liquid fuel synthesis unit 110.

### Liquid Fuel Synthesis Method

Next, a liquid fuel synthesis method using the liquid fuel synthesis system 100 will be described.

The liquid fuel synthesis method includes a step of supplying the raw material gas to the non-permeation side of the separation membrane 112 and supplying the sweep gas containing hydrogen or carbon dioxide as the main component to the permeation side of the separation membrane 112. On the non-permeation side of the separation membrane 112, the conversion reaction of the raw material gas to the liquid fuel is promoted. On the permeation side of the separation membrane 112, water vapor that has permeated through the separation membrane 112 is taken into the sweep gas.

The liquid fuel synthesis method further includes a step of removing moisture from the discharged gas containing the sweep gas and water vapor. In the present embodiment, in this step, the material gas is used as the refrigerant. By doing so, the material gas can be heated and the discharged gas can be cooled at the same time, and thus heat efficiency in the liquid fuel synthesis system 100 can be improved.

The liquid fuel synthesis method further includes a step of pressurizing the mixed gas in which the material gas that has been used as the refrigerant and the sweep gas are mixed. In this step, it is preferable that the raw material gas is generated by mixing the residual raw material gas separated from the liquid fuel with the mixed gas. In this manner, utilization efficiency of the raw material gas can be improved.

### Modification of Embodiment

Although an embodiment of the present invention has been described above, the present invention is not limited to the above embodiment, and various modifications can be made without departing from the gist of the invention.

### Modification 1

Although the catalyst layer 111 is disposed on the separation membrane 112 in the above embodiment, the present invention is not limited thereto. For example, the non-permeation side space 110A may be filled with a particulate catalyst. Although there is no limitation on the particle size (diameter) of the particulate catalyst, it can be 0.5 mm or more and 10 mm or less, for example.

### Modification 2

In the above embodiment, although the liquid fuel synthesis system 100 includes the liquid fuel synthesis unit 110 which is the membrane reactor, the present invention is not limited thereto.

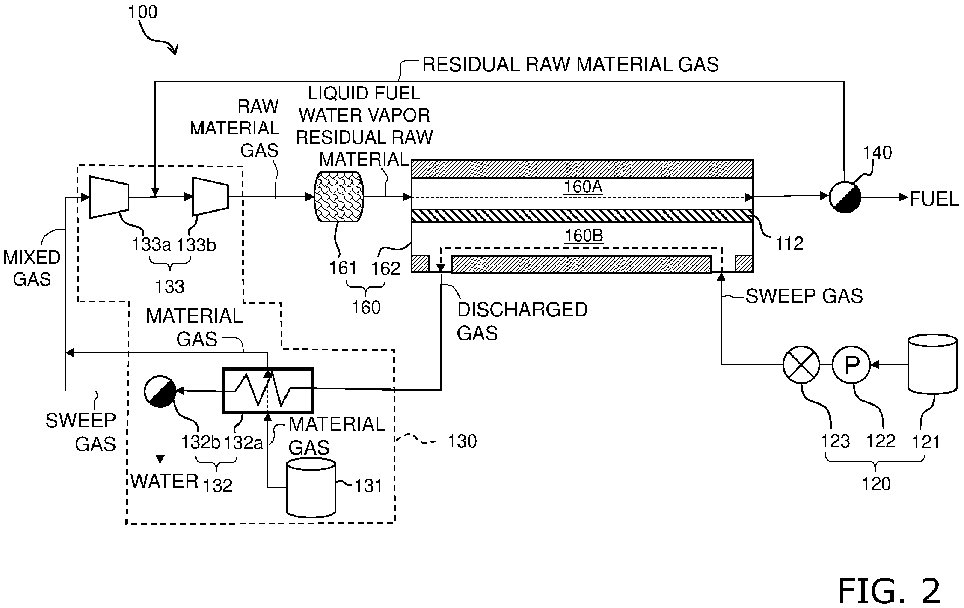

For example, as shown in FIG. 2, the liquid fuel synthesis system 100 may include the liquid fuel synthesis unit 160 having the catalyst unit 161 and the separation portion 162.

The catalyst unit 161 is supplied with the raw material gas from the raw material gas supply unit 130. The catalyst described in the above embodiment is disposed in the catalyst unit 161. The catalyst unit 161 converts the raw material gas to the liquid fuel.

The separation unit 162 includes the separation membrane 112, the non-permeation side space 160A, and the permeation side space 160B.

The liquid fuel, water vapor, and the residual fuel gas flow into the non-permeation side space 160A. Water vapor permeates through the separation membrane 112. Also, some of the hydrogen included in the residual fuel gas permeates through the separation membrane 112. The liquid fuel flows out from the non-permeation side space 160A without permeating through the separation membrane 112.

Water vapor that has permeated through the separation membrane 112 flows into the permeation side space 160B. The sweep gas supplied from the sweep gas supply unit 120 flows into the permeation side space 160B. The discharged gas containing the sweep gas, water vapor, and the hydrogen flows out from the permeation side space 160B.

In this modification as well, due to the sweep gas containing hydrogen or carbon dioxide as the main component, hydrogen that permeates through the separation membrane 112 can be reused as part of the raw material gas without separating from the sweep gas. As a result, the utilization efficiency of the raw material gas can be easily improved.

### Modification 3

Although the raw material gas and the sweep gas flow in opposite directions (that is, opposing directions) as seen in the side view of the separation membrane 112 in FIGS. 1 and 2, the raw material gas and the sweep gas may flow in the same direction (that is, parallel directions).

### Modification 4

Although the material gas supplied from the material gas source 131 is used as the refrigerant of the heat exchanger 132a in the above embodiment, the present invention is not limited thereto. Water or the like may be used as the refrigerant of the heat exchanger 132a. In this case, the material gas may be directly mixed with the sweep gas flowing out from the second drain trap 132b without passing through the heat exchanger 132a.

### Modification 5

Although all of the residual raw material gas separated from the liquid fuel in the first drain trap 140 is returned to the raw material gas supply unit 130 in the above embodiment, the present invention is not limited thereto.

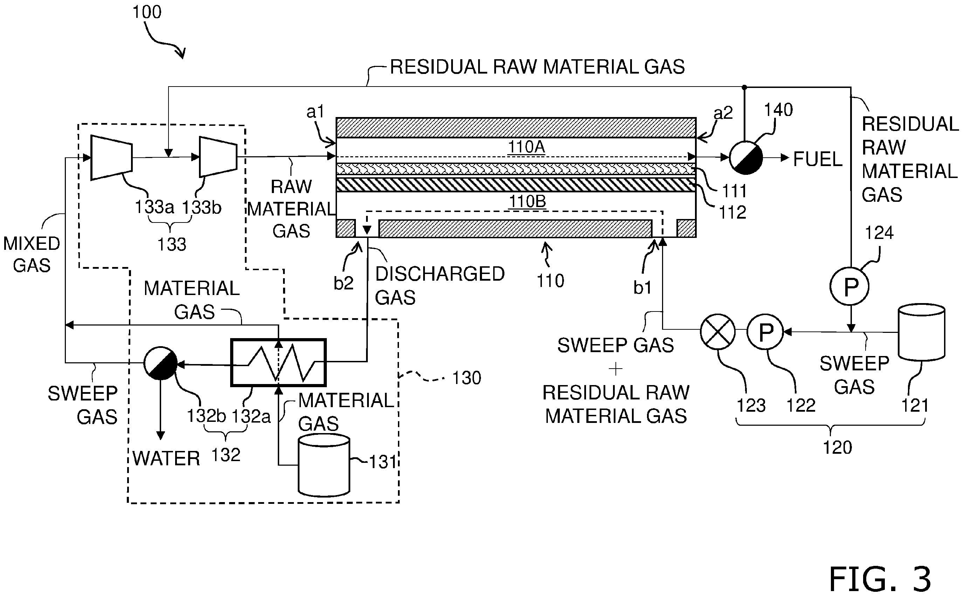

For example, as shown in FIG. 3, part of the residual raw material gas may be mixed with the sweep gas flowing out from the storage unit 121 and supplied to the flow rate adjustment mechanism 122. In this case, the part of the residual raw material gas is used as part of the sweep gas. The mixed amount of the residual raw material gas can be adjusted by the flow rate adjustment mechanism 124.

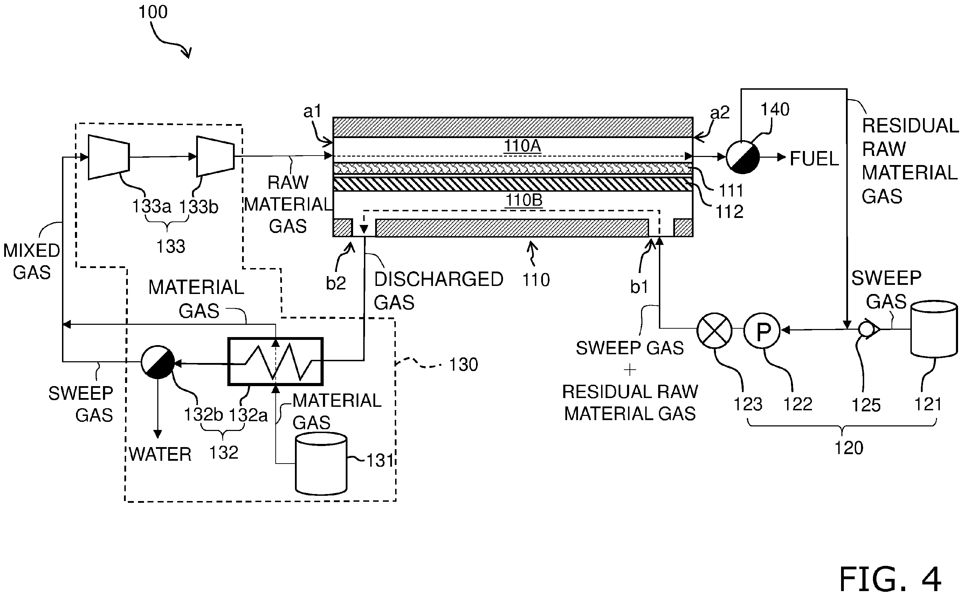

Also, as show in FIG. 4, all of the residual raw material as may be mixed with the sweep gas flowing out from the storage unit 121 and supplied to the flow rate adjustment mechanism 122. The residual raw material gas flows toward the flow rate adjustment mechanism 122 because a check valve 125 restricts it from flowing toward the storage unit 121. In this case, all of the residual raw material is used as part of the sweep gas.

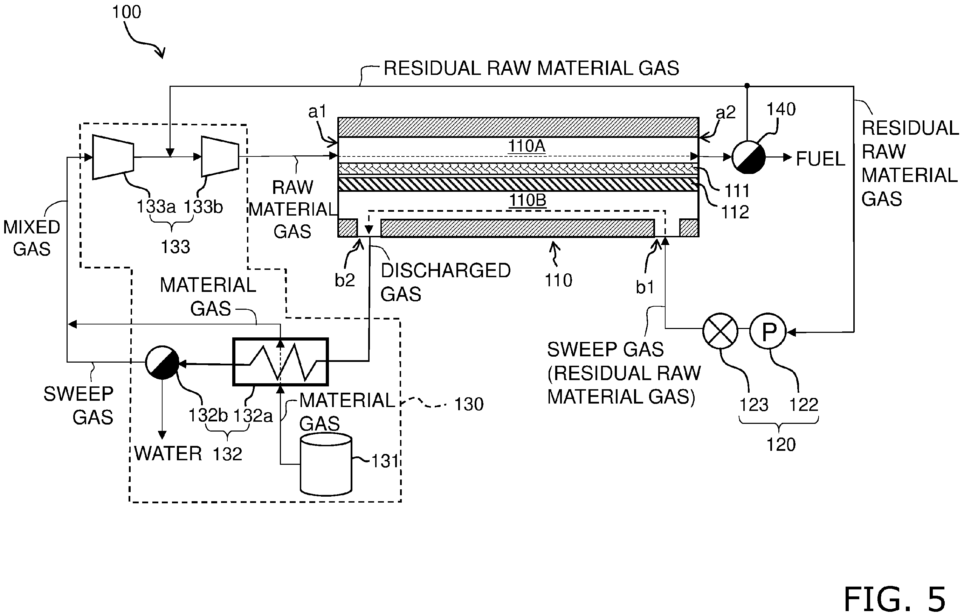

Alternatively, as shown in FIG. 5, the sweep gas supply unit 120 may not have the storage unit 121 and part of the residual raw material gas may be supplied to the flow rate adjustment mechanism 122. In this case, part of the residual raw material gas is used as it is as the sweep gas. A supply amount of the sweep gas (residual raw material gas) can be adjusted by the flow rate adjustment mechanism 122.

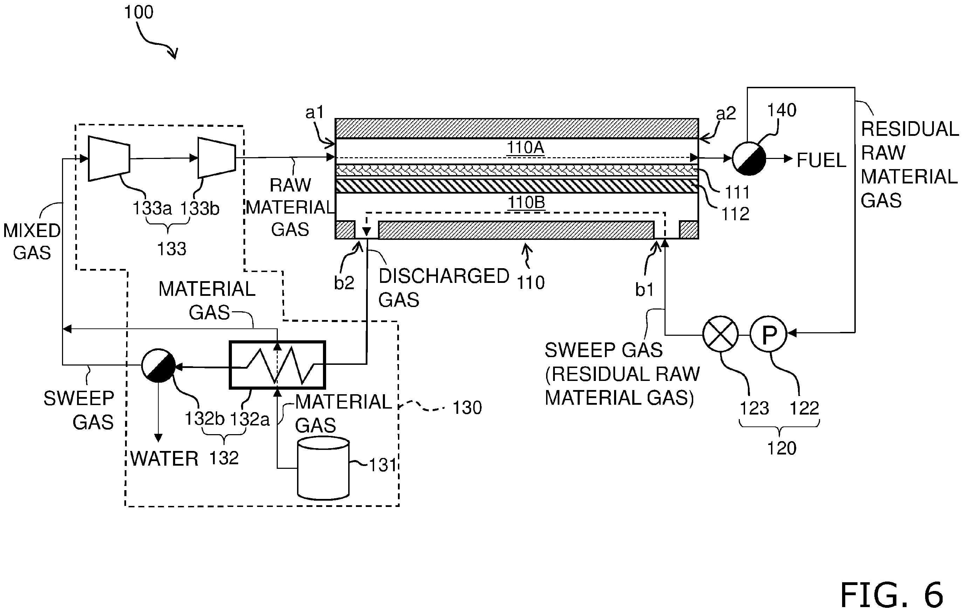

Alternatively, as shown in FIG. 6, the sweep gas supply unit 120 may not have the storage unit 121 and all of the residual raw material gas may be supplied to the flow rate adjustment mechanism 122. In this case, all of the residual raw material gas is used as it is as the sweep gas.

### Modification 6

Although the separation membrane 112 is permeable to water vapor that is a product of the conversion reaction of the raw material gas to the liquid fuel in the above embodiment, the present invention is not limited thereto. The separation membrane 112 may be permeable to the liquid fuel itself, generated through the conversion reaction of the raw material gas to the liquid fuel. In this case as well, the reaction equilibrium of the above formula (1) can be shifted to the product side.

Also, when the separation membrane 112 is permeable to the liquid fuel, even when generating the liquid fuel through a reaction in which no water vapor is generated (e.g., 2H₂ + CO ⇔ CH₃OH), the reaction equilibrium can be shifted to the product side.

### REFERENCE SIGNS LIST

- 1: Membrane reactor
- 100: Liquid fuel synthesis system
- 110: Liquid fuel synthesis unit
- 111: Catalyst layer
- 112: Separation membrane
- 110A: Non-permeation side space
- 110B: Permeation side space
- 120: Sweep gas supply unit
- 130: Raw material gas supply unit

## Claims

1. A liquid fuel synthesis system comprising:
a liquid fuel synthesis unit having a separation membrane permeable to a product of a conversion reaction of a raw material gas containing at least hydrogen and carbon dioxide to a liquid fuel; and
a sweep gas supply unit configured to supply a sweep gas for sweeping the product permeating through the separation membrane to the liquid fuel synthesis unit,
the sweep gas containing hydrogen or carbon dioxide as a main component.

2. The liquid fuel synthesis system according to claim 1, wherein
the sweep gas contains hydrogen as a main component.

3. The liquid fuel synthesis system according to claim 2, wherein
the sweep gas contains carbon dioxide as a secondary component.

4. The liquid fuel synthesis system according to any one of claims 1 to 3, comprising
a moisture removal unit configured to remove moisture from a discharged gas discharged from the liquid fuel synthesis unit, the discharged gas containing the sweep gas and the product.

5. The liquid fuel synthesis system according to claim 4, wherein
the moisture removal unit includes a heat exchanger using a material gas containing at least hydrogen and carbon dioxide, as a refrigerant.

6. The liquid fuel synthesis system according to claim 5, comprising
a pressurizing unit configured to pressurize a mixed gas in which the material gas passing through the moisture removal unit and the sweep gas are mixed, wherein
the pressurizing unit is configured to supply the mixed gas to the liquid fuel synthesis unit.

7. A liquid fuel synthesis method comprising
a step of supplying a sweep gas for sweeping a product to a permeation side of a separation membrane while promoting a conversion reaction of a raw material gas containing at least hydrogen and carbon dioxide to a liquid fuel by supplying the raw material gas to a non-permeation side of the separation membrane, the product being generated through the conversion reaction and permeating through the separation membrane, wherein
the sweep gas contains hydrogen or carbon dioxide as a main component.

8. The liquid fuel synthesis method according to claim 7, further comprising
a step of removing moisture from a discharged gas containing the sweep gas and the product.

9. The liquid fuel synthesis method according to claim 8, wherein
in the step of removing moisture from the discharged gas, a material gas containing at least hydrogen and carbon dioxide is used as a refrigerant.

10. The liquid fuel synthesis method according to claim 9, further comprising
a step of pressurizing a mixed gas in which the material gas used as the refrigerant and the sweep gas are mixed.
